(19) Europäisches Patentamt

European Patent Office

Office européen des brevets

(11) Veröffentlichungsnummer: **0 241 865**

**A2**

(12) **EUROPÄISCHE PATENTANMELDUNG**

(21) Anmeldenummer: 87105263.5

(22) Anmeldetag: 09.04.87

(51) Int. Cl.4: **C12M 3/00 , C12M 3/02**

(30) Priorität: **14.04.86 DE 3612453**

(43) Veröffentlichungstag der Anmeldung:
**21.10.87 Patentblatt 87/43**

(84) Benannte Vertragsstaaten:
**CH DE FR GB LI NL SE**

(71) Anmelder: **Diessel GmbH & Co.
Steven 1
D-3200 Hildesheim-Bavenstedt(DE)**

Anmelder: **Gesellschaft für
Biotechnologische Forschung mbH (GBF)
Mascheroder Weg 1
D-3300 Braunschweig-Stöckheim(DE)**

(72) Erfinder: **Lehmann, Jürgen, Dr.-Ing.
Bolkenhain 2 d
D-3300 Braunschweig(DE)**

(74) Vertreter: **Wehser, Wulf, Dipl.-Ing. et al
Patentanwälte Wehser & Fleuchaus
Roscherstrasse 12
D-3000 Hannover 1(DE)**

(54) **Vorrichtung zur Kultivierung von Zellkulturen.**

(57) Eine Vorrichtung zur Kultivierung von Zellkulturen, bei welcher die Kulturen sich innerhalb wenigstens eines flüssigen Nährmediums in einem geschlossenen Behälter befinden, wobei das Nährmedium oder die Nährmedien dem Behälter über wenigstens eine durch eine Pumpe beaufschlagte Zufuhrleitung zugeführt werden, und wobei dem oberhalb des Flüssigkeitsspiegels befindlichen Kopfraum des geschlossenen Behälters ein Gas (Sauerstoff, stickstoff oder Luft od. dgl.) zugeführt wird, dessen Druck im Kopfraum konstant gehalten wird, soll so ausgebildet werden, daß die Schädigung von zwangsweise austretenden freien Zellen bei der Abfuhr der Nährmedien wirksam verhindert ist.

Hierzu ist erfindungsgemäß vorgesehen, daß eine mit der Flüssigkeit im Behälter in Verbindung stehende außerhalb des Behälters endende Überlaufleitung vorgesehen ist, deren freies Ende so weit oberhalb des Flüssigkeitsspiegels im Behälter liegt, daß sich der durch die Flüssigkeitssäule in der Überlaufleitung erzeugte Druck im Gleichgewicht mit dem Gasdruck im Kopfraum des Behälters befindet.

FIG.1

$\Delta P = P_{Kopfraum}$

## Vorrichtung zur Kultivierung von Zellkulturen

Die Erfindung betrifft eine Vorrichtung zur Kultivierung von Zellkulturen, bei welcher die Kulturen sich innerhalb wenigstens eines flüssigen Nährmediums in einem geschlossenen Behälter befinden, wobei das Nährmedium oder die Nährmedien dem Behälter über wenigstens eine durch eine Pumpe beaufschlagte Zufuhrleitung zugeführt werden, und wobei dem oberhalb des Flüssigkeitsspiegels befindlichen Kopfraum des geschlossenen Behälters ein Gas (Sauerstoff, Stickstoff oder Luft od. dgl.) zugeführt wird, dessen Druck im Kopfraum konstant gehalten wird.

Die Zufuhr der Nährmedien ist erforderlich, weil in zeitlichen Abschnitten das Nährmedium im Behälter ersetzt werden muß. Gleichzeitig mit der Zufuhr muß also für die Abfuhr der überschüssigen Flüssigkeit Sorge getragen werden.

Hierzu ist es bereits bekannt, in eine Abfuhrleitung eine Pumpe einzusetzen, die so viel Flüssigkeit aus dem Behälter abfördert, wie diesem über die Nährmedienzuleitungen zugeführt wird. In diesem Zusammenhang ist es bereits bekannt, die in den Zufuhrleitungen und in der Abfuhrleitung angeordneten Pumpen mittels einer Niveauregulierung so zu steuern, daß die abgeförderte Flüssigkeitsmenge immer möglichst genau derjenigen entspricht, die zugeführt wird.

Für eine solche Niveauregulierung ist es bereits bekannt, entweder mit einer Sonde den Flüssigkeitsspiegel abzutasten, oder aber den Behälter zu wiegen und die so ermittelten Werte dem Ein-und Abschalten der Pumpen zugrunde zu legen.

Nachteilig bei allen diesen bekannten Anordnungen ist es, daß bei einer Verwendung einer Pumpe in der Abfuhrleitung die mit der Nährmittelflüssigkeit austretenden freien Zellen durch die Pumpe beschädigt oder zerstört werden können, wobei diese beschädigten Zellen das zu erzielende Produkt (biologische Moleküle, Proteine usw.) abbauen können, so daß entweder das Produkt überhaupt nicht erreicht wird oder daß die Erzeugung des Produktes unwirtschaftlich wird.

Der Erfindung liegt demgemäß die Aufgabe zugrunde, eine Vorrichtung der eingangs genannten Art so auszubilden, daß die Nachteile der bekannten Anordnungen vermieden werden und daß insbesondere die Schädigung der zwangsweise austretenden, an sich unerwünschten freien Zellen wirksam verhindert ist.

Diese Aufgabe wird erfindungsgemäß dadurch gelöst, daß eine mit der Flüssigkeit im Behälter in Verbindung stehende außerhalb des Behälters endende Überlaufleitung vorgesehen ist, deren freies Ende so weit oberhalb des Flüssigkeitsspiegels im Behälter liegt, daß sich der durch die Flüssigkeitssäule in der Überlaufleitung erzeugte Druck im Gleichgewicht mit dem Gasdruck im Kopfraum des Behälters befindet.

Mit dieser Anordnung wird erreicht, daß zur Abförderung Pumpen oder andere mechanische Teile nicht mehr erforderlich sind, weil durch die unter Druck zugeführten Nährmedien der Gasdruck im Kopfraum des Behälters erhöht wird, so daß die zugeführte Menge automatisch über die Überlaufleitung wieder abgeführt wird. Damit ist eine Beschädigung der austretenden freien Zellen, die jetzt lediglich an den Leitungsinnenwandungen entlanggleiten müssen, praktisch ausgeschlossen.

Besonders zweckmäßig ist es, wenn das freie Ende der Überlaufleitung in ein offenes Überlaufgefäß mündet, das seinerseits einen Auslaufstutzen aufweist, der über eine Abflußleitung mit einem das Produkt aufnehmenden Aufnahmebehälter in Verbindung steht.

Das Überlaufgefäß muß offen sein, damit sich in diesem und in der Abflußleitung kein Druck aufbaut, welcher den Abfluß verhindern würde. Zweckmäßigerweise ist an die Öffnung des Überlaufgefäßes eine Zweigleitung angeschlossen, in welcher sich ein Sterilfilter befindet, wobei die Zweigleitung jenseits des Sterilfilters offen ist.

Vorteilhaft ist es weiter, wenn sowohl die Überlaufleitung als auch die Abflußleitung zum Aufnahmebehälter wenigstens abschnittsweise flexibel sind, da hierdurch die Möglichkeit besteht, durch Zusammendrücken der Leitungen von Hand eventuelle Staus od. dgl. zu beseitigen, ohne daß hierdurch die freien Zellen beschädigt werden können.

Das freie Ende der Überlaufleitung kann höhenverstellbar ausgebildet sein, wodurch die Möglichkeit besteht, das Druckniveau zu ändern und damit die Menge des zuzuführenden Mediums. Dies hat den erheblichen Vorteil, daß der Druck im Kopfraum durch das Verschieben des freien Endes der Überlaufleitung frei wählbar ist. Dies hat insbesondere Bedeutung, wenn über Membranen die im Behälter enthaltene Flüssigkeit begast werden soll, da die Ausbildung der Gasblasen in den Poren der Membran vom Außendruck außerhalb der Membran abhängt.

Das behälterseitige Ende der Überlaufleitung kann direkt in die im Behälter enthaltene Flüssigkeit jenseits des Flüssigkeitsspiegels eintauchen; es ist aber auch möglich, eine an den Behälter in dessen unterem Bereich angeschlossene Zweigleitung zu verwenden, die unterhalb des Flüssigkeitsspiegels endet, wobei das behälterseitige Ende der Überlaufleitung an diese Zweigleitung angeschlossen ist. Die Niveaudifferenz zwischen dem Flüssigkeitsspiegel und dem freien Ende der Überlaufleitung wird hierdurch nicht berührt.

Zweckmäßigerweise wird der Gasdruck im Kopfraum mittels eines in einer Abfuhrleitung angeordneten Druckhalteventils konstant gehalten.

Die Erfindung wird im folgenden anhand von Ausführungsbeispielen in der Zeichnung näher erläutert.

Fig. 1 zeigt in schematischer Darstellung eine Ausführungsform der erfindungsgemäßen Vorrichtung.

Fig. 2 zeigt den Behälter aus Fig. 1 bei einer abgewandelten Ausführungsform.

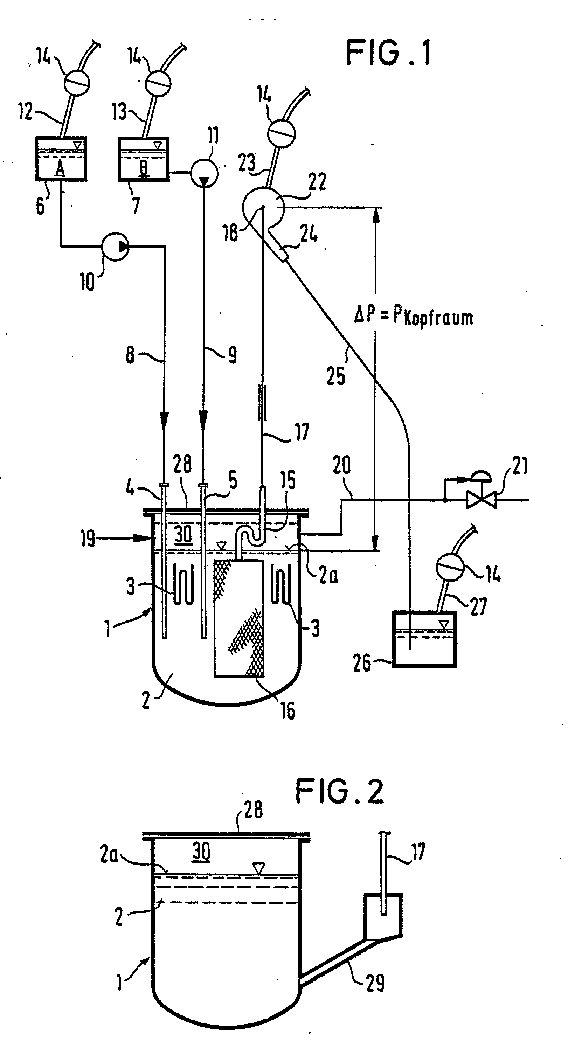

Gemäß Fig. 1 ist ein Behälter 1 vorgesehen, in welchem sich in einer Nährmittelflüssigkeit 2 Zellkulturen befinden, die kultiviert werden sollen. Hierzu kann beispielweise über Membranschläuche 3 die Flüssigkeit 2 begast werden.

Da die Nährmittelflüssigkeit 2 im Behälter 1 von Zeit zu Zeit ersetzt werden muß, sind in die Flüssigkeit eintauchende Rohre 4 und 5 vorgesehen, über welche in offenen Vorratsbehältern 6 und 7 enthaltene Nähremedien A und B der Flüssigkeit 2 im Behälter 1 zugeführt werden können. Diese Zufuhr geschieht unter Druck, der durch in Zufuhrleitungen 8 und 9 angeordnete Schlauchpumpen 10 und 11 aufgebracht wird.

Die Vorratsbehälter 6 und 7 stehen über Zweigleitungen 12 und 13 mit der Atmosphäre in Verbindung, wobei in die Zweigleitungen Sterilfilter 14 eingeschaltet sind.

Für die Flüssigkeitsabfuhr ist ein schwanenhalsförmiges Schlauchstück 15 vorgesehen, welches mit seinem unteren Ende ebenfalls in die Flüssigkeit 2 eintaucht, wobei ein Sieb 16 zwischen dem eintauchenden Ende des Schlauchstückes 15 und der Flüssigkeit 2 innerhalb dieser angeordnet ist. An das Schlauchstück 15 schließt eine Überlaufleitung 17 an, deren freies Ende 18 so weit oberhalb des Flüssigkeitsspiegels 2a im Behälter 1 liegt, daß sich der durch die Flüssigkeitssäule in der Überlaufleitung 17 erzeugte Druck im Gleichgewicht mit dem Druck im Kopfraum 30 des Behälters 1 befindet.

Der Druck im Kopfraum 30 des Behälters 1 wird durch die Zufuhr eines Gases (Sauerstoff, Stickstoff oder Luft od. dgl.) erzeugt, wobei in Fig. 1 die Gaszufuhr durch den Pfeil 19 angedeutet ist. Der so erzeugte Druck im Kopfraum 30 wird mittels eines in einer Abfuhrleitung 20 angeordneten Druckhaltesventiles 21 konstant gehalten.

Das freie Ende 18 der Überlaufleitung 17 mündet, wie dargestellt, in ein offenes Überlaufgefäß 22, dessen Öffnung bei der dargestellten Ausführungsform mit einer Zweigleitung 23 in Verbindung steht, in welche wiederum ein Sterilfilter 14 eingeschaltet ist.

Das Überlaufgefäß 22 hat einen Auslaufstutzen 24, der über eine Abflußleitung 25 mit einem das Produkt aufnehmenden Aufnahmebehälter 26 in Verbindung steht. Auch dieser Behälter 26 ist über eine Zweigleitung 27 mit eingeschaltetem Sterilfilter 14 offen.

Das freie Ende 18 der Überlaufleitung ist vorteilhafterweise entweder innerhalb des Überlaufgefäßes 22 oder zusammen mit diesem höhenverstellbar. Auf diese Weise ist der Druck im Kopfraum 30 des Behälters 1 einstell-und wählbar.

Die Wirkungsweise der beschriebenen Anordnung ist folgende:

Durch die Gaszufuhr in den Kopfraum 30 des mittels eines Deckels 28 verschlossenen Behälters 1 wird in dem Kopfraum 30 ein durch das Gas gebildetes Druckpolster erzeugt, das durch das Druckhalteventil 21 kontant gehalten wird. Wenn jetzt aus den Vorratsbehältern 6 und/oder 7 durch die Kraft der Pumpen 10 und/oder 11 Nährmedium über die Leitungen 8 und 9 gegen den im Kopfraum 30 herrschenden Druck zugeführt wird, führt dies zu einer Druckerhöhung im Kopfraum 30, welcher die Flüssigkeitssäule in der Überlaufleitung 17 nach oben verschiebt, so daß die über die Leitungen 8 und/oder 9 zugeführte Flüssigkeitsmenge am freien Ende 18 der Überlaufleitung 17 in das Überlaufgefäß 22 austritt und von dort zum Aufnahmebehälter 26 geführt wird. Die Flüssigkeitsabfuhr erfolgt also ohne die Zuhilfenahme bewegter mechanischer Teile, so daß die austretenden Zellen nicht beschädigt werden können.

Fig. 2 zeigt eine etwas abgewandelte Ausführungsform, bei welcher an den Behälter 1 eine Zweigleitung 29 unterhalb des Flüssigkeitsspiegels 2a angeschlossen ist, wobei das behälterseitige Ende der Überlaufleitung 17 mit dieser Zweigleitung in Verbindung steht. Gleichwohl bleibt die Niveaudifferenz zwischen dme Flüssigkeitsspiegel 2a und dem freien Ende 18 der Überlaufleitung 17 für die jeweils abzufördernde Flüssigkeitsmenge maßgebend.

**Ansprüche**

1. Vorrichtung zur Kultivierung von Zellkulturen, bei welcher die Kulturen sich innerhalb wenigstens eines flüssigen Nährmediums in einem geschlossenen Behälter befinden, wobei das Nährmedium oder die Nährmedien dem Behälter über wenigstens eine durch eine Pumpe beaufschlagte Zufuhrleitung zugeführt werden, und wobei dem oberhalb des Flüssigkeitsspiegels befindlichen Kopfraum des geschlossenen Behälters ein Gas (Sauerstoff, Stickstoff oder Luft od. dgl.) zugeführt wird, dessen Druck im Kopfraum konstant gehalten wird, dadurch gekennzeichnet, daß eine mit der Flüssigkeit (2) im Behälter (1) in Verbindung stehende außerhalb des Behälters (1) endende Überlaufleitung (17) vorgesehen ist, deren freies Ende (18) so weit oberhalb des Flüssigkeitsspiegels (2a) im Behälter (1) liegt, daß sich der durch die Flüssigkeitssäule in der Überlaufleitung (17) erzeugte Druck im Gleichgewicht mit dem Gasdruck im Kopfraum (30) des Behälters (1) befindet.

2. Vorrichtung nach Anspruch 1, dadurch gekennzeichnet daß das freie Ende (18) der Überlaufleitung (17) in ein offenes Überlaufgefäß - (22) mündet, das seinerseits einen Auslaufstutzen (24) aufweist, der über eine Abflußleitung (25) mit einem das Produkt aufnehmenden Aufnahmebehälter (26) in Verbindung steht.

3. Vorrichtung nach Anspruch 2, dadurch gekennzeichnet, daß an die Öffnung des Überlaufgefäßes (22) eine Zweigleitung (23) angeschlossen ist, in welcher sich ein Sterilfilter (14) befindet, wobei die Zweigleitung (23) jenseits des Sterilfilters (14) offen ist.

4. Vorrichtung nach einem der vorhergehenden Ansprüche, dadurch gekennzeichnet, daß sowohl die Überlaufleitung (17) als auch die Abflußleitung (25) zum Aufnahmebehälter (26) wenigstens abschnittsweise flexibel ausgebildet sind.

5. Vorrichtung nach einem der vorhergehenden Ansprüche, dadurch gekennzeichnet, daß das freie Ende (18) der Überlaufleitung (17) höhenverstellbar ausgebildet ist.

6. Vorrichtung nach Anspruch 5, dadurch gekennzeichnet, daß das freie Ende (18) der Überlaufleitung (17) innerhalb des Überlaufgefäßes (22) höhenverstellbar ist.

7. Vorrichtung nach Anspruch 5, dadurch gekennzeichnet, daß das freie Ende (18) zusammen mit dem Überlaufgefäß (22) höhenverstellbar ist.

8. Vorrichtung nach einem der vorhergehenden Ansprüche, dadurch gekennzeichnet, daß das behälterseitige Ende der Überlaufleitung (17) direkt in die im Behälter (1) enthaltene Flüssigkeit (2) jenseits des Flüssigkeitsspiegels (2a) eintaucht.

9. Vorrichtung nach einem der Ansprüche 1 bis 7, dadurch gekennzeichnet, daß an den Behälter (1) in dessen unterem Bereich eine Zweigleitung (29) angeschlossen ist, die unterhalb des Flüssigkeitsspiegels (2a) endet, wobei das behälterseitige Ende der Überlaufleitung (17) an diese Zweigleitung (29) angeschlossen ist.

10. Vorrichtung nach einem der vorhergehenden Ansprüche, dadurch gekennzeichnet, daß der Gasdruck im Kopfraum (30) mittels eines in einer Abfuhrleitung (20) angeordneten Druckhalteventils (21) konstant gehalten wird.

FIG.1

$\Delta P = P_{Kopfraum}$

FIG.2